# EUROPÄISCHE PATENTSCHRIFT

(11) **EP 1 566 442 B1**
(45) Veröffentlichungstag und Bekanntmachung des Hinweises auf die Patenterteilung: **25.11.2009**
(21) Anmeldenummer: 04008412.1
(22) Anmeldetag: 28.01.1991
(51) Int. Cl.: C12N 15/63, C12P 21/08, C07K 16/06, C12Q 1/68, G01N 33/53

(54) **Herstellung und Verwendung von Genbanken menschlicher Antikörper("Human-Antikörper-Bibliotheken")**
Preparation and use of a human antibody gene bank (human antibody libraries)
Préparation et utilisation de banques de gènes d'anticorps humains (bibliothèques d'anticorps humains)

(30) Priorität: 01.02.1990 DE 4002898; 09.02.1990 DE 4003881
(43) Veröffentlichungstag der Anmeldung: 24.08.2005
(62) Teilanmeldung aus: 91101095.7
(73) Patentinhaber: Siemens Healthcare Diagnostics Products GmbH, 35041 Marburg (DE)
(72) Erfinder: Little, Melvyn, Prof. Dr., 69151 Neckargemünd (DE); Breitling, Frank, Dr., 69115 Heidelberg (DE); Seehaus, Thomas, Dr., 64646 Heppenheim (DE); Dübel, Stefan, Prof. Dr., 38106 Braunschweig (DE); Klewinghaus, Iris, 68165 Mannheim (DE)

(56) Entgegenhaltungen:
- EP-A- 0 368 684
- HUSE W D ET AL: "GENERATION OF A LARGE COMBINATORIAL LIBRARY OF THE IMMUNOGLOBULIN REPERTOIRE IN PHAGE LAMBDA" PROCEEDINGS OF THE SPIE, SPIE, BELLINGHAM, VA, US, Bd. 246, 8. Dezember 1989 (1989-12-08), Seiten 1275-1281, XP001019235 ISSN: 0277-786X
- ORLANDI R ET AL: "CLONING IMMUNOGLOBULIN VARIABLE DOMAINS FOR EXPRESSION BY THE POLYMERASE CHAIN REACTION" PROCEEDINGS OF THE NATIONAL ACADEMY OF SCIENCES OF USA, NATIONAL ACADEMY OF SCIENCE, WASHINGTON, DC, US, Bd. 86, Nr. 10, 1. Mai 1989 (1989-05-01), Seiten 3833-3837, XP000026475 ISSN: 0027-8424
- WARD E S ET AL: "BINDING ACTIVITIES OF A REOPERTOIRE OF SINGLE IMMUNOGLOBULIN VARIABLE DOMAINS SECRETED FROM ESCHERICHIA COLI" NATURE, NATURE PUBLISHING GROUP, LONDON, GB, Bd. 341, Nr. 6242, 12. Oktober 1989 (1989-10-12), Seiten 544-546, XP000086104 ISSN: 0028-0836
- LEBOEUF R D ET AL: "CLONING AND SEQUENCING OF IMMUNOGLOBULIN VARIABLE-REGION GENES USING DEGENERATE OLIGODEOXYRIBONUCLEOTIDES AND POLYMERASE CHAIN REACTION" GENE (AMSTERDAM), Bd. 82, Nr. 2, 1989, Seiten 371-378, XP002375035 ISSN: 0378-1119
- YAWEN L CHIANG: "DIRECT CDNA CLONING OF THE REARRANGED IMMUNOGLOBULIN VARIABLE REGION" BIOTECHNIQUES, INFORMA LIFE SCIENCES PUBLISHING, WESTBOROUGH, MA, US, Bd. 7, Nr. 4, Januar 1989 (1989-01), Seiten 360-362,364, XP000350904 ISSN: 0736-6205

### SEQUENCE LISTING

<110> Dade Behring Marburg GmbH
<120> Preparation and Use of Gene Banks of Human Antibodies ("Human-Antibody-Libraries")
<130> 1990/B005 - Ma 833
<140> EP 04008412.1
   <141> 1991-01-28
<150> EP91101095.7
   <151> 1991-01-28
<160> 29
<170> PatentIn version 3.1
<210> 1
   <211> 32
   <212> DNA
   <213> Homo sapiens
<400> 1
   gaggtgcagc tgcaggagtc tgggggaggc tt 32
<210> 2
   <211> 37
   <212> DNA
   <213> Homo sapiens
<400> 2
   tgtctgcatc tgtrggagac agggtcacca tcamttg 37
<210> 3
   <211> 44
   <212> DNA
   <213> Homo sapiens
<400> 3
   cctcagygtc tgggwcccca ggacagaggg tgaccatctc ctgc 44
<210> 4
   <211> 45
   <212> DNA
   <213> Homo sapiens
<400> 4
   gggtgggacg aagaagctta cttagggagg cagctcagca atcac 45
<210> 5
   <211> 45
   <212> DNA
   <213> Homo sapiens
<400> 5
   gggtgggacg aagaagctaa gcttgggagg cagctcagca atcac 45
<210> 6
   <211> 61
   <212> DNA
   <213> Homo sapiens
<400> 6
   ggcacttcgg atcctaacac tctcccctgt tgaagctctt tgtgacgggc gagctcaggc 60
   c 61
<210> 7
   <211> 42
   <212> DNA
   <213> Homo sapiens
<400> 7
   gtgagggwtg ggatcctatg aacattctgt aggggccact gt 42
<210> 8
   <211> 43
   <212> DNA
   <213> Homo sapiens
<400> 8
   cacaggagac gagggggaaa agctttgggg cttatgcact ccc 43
<210> 9
   <211> 43
   <212> DNA
   <213> Homo sapiens
<400> 9
   cacaggagac gagggggaaa agctttgggg cggatgcact ccc 43
<210> 10
   <211> 54
   <212> DNA
   <213> Homo sapiens
<400> 10
   aacagaggcg gatcctcatt tcaactgctc atcagatggc gggaagatga agac 54
<210> 11
   <211> 39
   <212> DNA
   <213> Homo sapiens
<400> 11
   agctcctcag aggasggcgg gatccgagtg acctagggg 39
<210> 12
   <211> 27
   <212> PRT
   <213> Homo sapiens
<400> 12
   Met Lys Tyr Leu Leu Pro Thr Ala Ala Ala Gly Leu Leu Leu Leu Ala
   1 5 10 15
   Ala Gln Pro Ala Met Ala Gln Val Gln Leu Gln
   20 25
<210> 13
   <211> 82
   <212> DNA
   <213> Homo sapiens
<400> 13
   catgaaatac ctcttgccta cggcagccgc tggcttgctg ctgctggcag ctcagccggc 60
   gatggcgcaa gttcagctgc ag 82
<210> 14
   <211> 24
   <212> PRT
   <213> Homo sapiens
<400> 14
   Met Lys Tyr Leu Leu Pro Thr Ala Ala Ala Gly Leu Leu Leu Leu Ala
   1 5 10 15
   Ala Gln Pro Ala Met Ala Asp Ile
   20
<210> 15
   <211> 125
   <212> DNA
   <213> Homo sapiens
<400> 15
   ctgcagccaa gcttgaattc attaaagagg agaaattaac tccatgaagt acttactgcc 60
   gaccgctgcg gcgggtctcc tgctgttggc ggctcagccg gctatggctg atatcggatc 120
   cagct 125
<210> 16
   <211> 37
   <212> DNA
   <213> Homo sapiens
<400> 16
   catgaaatac ctcttgccta cggcagccgc tggcttg 37
<210> 17
   <211> 73
   <212> DNA
   <213> Homo sapiens
<400> 17
   gctgaacttg cgccatcgcc ggctgagctg ccagcagcag caagccagcg gctgccgtag 60
   gcaagaggta ttt 73
<210> 18
   <211> 44
   <212> DNA
   <213> Homo sapiens
<400> 18
   ctgctgctgg cagctcagcc ggcgatggcg caagttcagc tgca 44
<210> 19
   <211> 30
   <212> DNA
   <213> Homo sapiens
<400> 19
   gccaagcttg aattcattaa agaggagaaa 30
<210> 20
   <211>
   <212> DNA
   <213> Homo sapiens
<400> 20
   ttaactccat gaagtactta ctgccgaccg ctgcg 35
<210> 21
   <211> 124
   <212> DNA
   <213> Homo sapiens
<400> 21
   agctggatcc gatatcagcc atagccggct gagccgccaa cagcaggaga cccgccgcag 60
   cggtcggcag taagtacttc atggagttaa tttctcctct ttaatgaatt caagcttggc 120
   tgca 124
<210> 22
   <211> 30
   <212> DNA
   <213> Homo sapiens
<400> 22
   gctcagccgg ctatggctga tatcggatcc 30
<210> 23
   <211> 21
   <212> DNA
   <213> Homo sapiens
<400> 23
   gcgggtctcc tgctgttggc g 21
<210> 24
   <211> 28
   <212> DNA
   <213> Homo sapiens
<400> 24
   agcttgaaga aggtgaagaa ttctaatg 28
<210> 25
   <211> 28
   <212> DNA
   <213> Homo sapiens
<400> 25 28
   agctcattag aattcttcac cttcttca 28
<210> 26
   <211> 120
   <212> PRT
   <213> Homo sapiens
<400> 26
   Gly Val His Ser Gln Val Gln Leu Gln Glu Ser Gly Pro Gly Leu Val
   1               5                   10                  15
   Arg Pro Ser Gln Thr Leu Ser Leu Thr Cys Thr Val Ser Gly Phe Thr
               20                  25                  30
   Phe Ser Gly Tyr Gly Val Asn Trp Val Arg Gln Pro Pro Gly Arg Gly
           35                  40                  45
   Leu Glu Trp Ile Gly Met Ile Trp Gly Asp Gly Asn Thr Asp Tyr Asn
       50                  55                  60
   Ser Ala Leu Lys Ser Arg Val Thr Met Leu Val Asp Thr Ser Lys Asn
   65                  70                  75                  80
   Gln Phe Ser Leu Arg Leu Ser Ser Val Thr Ala Ala Asp Thr Ala Val
                   85                  90                  95
   Tyr Tyr Cys Ala Arg Glu Arg Asp Tyr Arg Leu Asp Tyr Trp Gly Gln
               100                 105                 110
   Gly Ser Leu Val Thr Val Ser Ser
           115                 120
<210> 27
   <211> 407
   <212> DNA
   <213> Homo sapiens
<400> 27
   ctctccacag gtgtccactc ccaggtccaa ctgcaggaga gcggtccagg tcttgtgaga     60
   cctagccaga ccctgagcct gacctgcacc gtgtctggct tcaccttcag cggctatggt    120
   gtaaactggg tgagacagcc acctggacga ggtcttgagt ggattggaat gatttggggt    180
   gatggaaaca cagactataa ttcagctctc aaatccagag tgacaatgct ggtagacacc    240
   agcaagaacc agttcagcct gagactcagc agcgtgacag ccgccgacac cgcggtctat    300
   tattgtgcaa gagagagaga ttataggctt gactactggg gtcagggctc cctcgtcaca    360
   gtctcctcat aagcttcctt acaacctctc tcttctattc agcttaa                  407
<210> 28
   <211> 113
   <212> PRT
   <213> Homo sapiens
<400> 28
   Gly Val His Ser Asp Ile Gln Asn Thr Gln Ser Pro Ser Ser Leu Ser
   1               5                   10                  15
   Ala Ser Val Gly Asp Arg Val Thr Ile Thr Cys Arg Ala Ser Gly Asn
               20                  25                  30
   Ile His Asn Tyr Leu Ala Trp Tyr Gln Gln Lys Pro Gly Lys Ala Pro
           35                  40                  45
   Lys Leu Leu Ile Tyr Tyr Thr Thr Thr Leu Ala Asp Gly Val Pro Ser
       50                  55                  60
   Arg Phe Ser Gly Ser Gly Ser Gly Thr Asp Phe Thr Phe Thr Ile Ser
   65                  70                  75                  80
   Ser Leu Gln Pro Glu Asp Ile Ala Thr Tyr Tyr Cys Gln His Phe Trp
                   85                  90                  95
   Ser Thr Pro Arg Thr Phe Gly Gln Gly Thr Lys Val Glu Ile Lys Arg
               100                 105                 110
   Glu
<210> 29
   <211> 381
   <212> DNA
   <213> Homo sapiens
<400> 29
   ctctccacag gtgtccactg ggatatccag atgacccaga gcccaagcag cctgagcgcc     60
   agcgtgggtg acagggtgac catcacctgt agagccagcg gtaacatcca caactacctg    120
   gcttggtacc agcagaagcc aggtaaggct ccaaagctgc tgatctacta caccaccacc    180
   ctggctgacg gtgtgccaag cagattcagc ggtagcggta gcggtaccga cttcaccttc    240
   accatcagca gcctccagcc agaggacatc gccacctact actgccagca cttctggagc    300
   accccaagga cgttcggcca aggtaccaag gtggaaatca aacgtgagta gaatttaaac    360
   tttgcttcct cagttggatc c                                              381

## Beschreibung

Die Erfindung betrifft die Herstellung und Verwendung von Genbanken menschlicher Antikörper (AK). Ausgehend von einem Gemisch humaner B-Lymphozyten wird deren mRNA, unter Verwendung von Oligo-dT-Primern in cDNA übersetzt. Anschließend findet eine Amplifikation der AK-spezifischen cDNA mittels Polymerase-Ketten Reaktion. (PCR, Polymerase Chain Reaction) unter Einsatz von geeigneten Oligonukleotid-Primersequenzen statt. Durch Expression , dieser amplifizierten AK-spezifischen cDNA in einem bakteriellen Expressionsvektor, z.B. dem nachstehend beschriebenen Vektor pFMT, in E.coli, steht somit eine Human-Antikörper-Bibliothek mit einem umfassenden Repertoire zum Durchprüfen ("Screenen") mit ausgewählten Antigenen in vitro zur Verfügung.

Es wird geschätzt, daß das Immunsystem des Menschen bzw. eines Säugetiers zwischen 10⁶ und 10⁸ verschiedene Antikörper besitzt. Diese Zahl von Antikörpern reicht aus, um eine Immunreaktion des Körpers sowohl gegen sämtliche in der Natur vorkommende Antigene als auch gegen künstliche Antigene hervorzurufen. Wenn man weiterhin in Betracht zieht, daß oft mehrere Antikörper mit demselben Antigen reagieren, wird das Repertoire an wirklich verschiedenen Antikörpern eher im Bereich 10⁶ bis 10⁷ anzusiedeln sein.

Bisher wurden spezifische Antikörper stets ausgehend von einer Immunisierung mit dem jeweiligen Antigen erhalten, beispielsweise Injektion des Antigens in den Organismus oder Inkubation von Milzzellen in vitro mit diesem Antigen. Im Fall von polyklonalen Antikörpern lassen sich die Immunglobuline anschließend aus dem Serum isolieren und z.B. durch Absorptiönsverfahren die spezifischen Antikörper daraus gewinnen. Monoklonale Antikörper werden aus den Zellüberständen bwz. dem Zell-Lysat von mit einzelnen B-Lymphozyten fusionierten, klonierten Milztumorzellen (Hybridomzellen) isoliert. Die oben geschilderten Verfahren sind insbesondere zur Herstellung von spezifischen Human-Antikörpern bzw. humanen monoklonalen Antikörpern nicht geeignet.

Die vorliegende Erfindung stellte sich deshalb die Aufgabe, eine allgemein gangbare Methode zur Erzeugung spezifischer humaner monoklonaler Antikörper (huMAK's) oder von Antikörperteilen, die die Antigenbindungsstelle enthalten, zu entwickeln.

Es wurde gefunden, daß die gesuchten huMAK's oder deren Teile, die die variable, antigenbindende Domäne enthalten, aus Genbanken humaner Immunglobline isoliert werden können. Zunächst wurde ausgehend von einem Gemisch nicht aktivierter humaner B-Lymphozyten deren mRNA isoliert und mit Hilfe von Oligo-dT-Primern in cDNA übersetzt. Eine spezifische Amplifizierung der Population von Antikörper cDNAs innerhalb des erhaltenen cDNA-Pools wurde durch die Verwendung der PCR erreicht. Dazu wurden bestimmte Oligonukleotid-"Primer" verwendet, die homolog zu konservierten Sequenzen an beiden Enden der Antikörper-cDNA sind (siehe unten und Beispiele). Der Entwurf der Primer für die Rückreaktion zur Synthese des nicht-kodierenden Stranges der DNA der schweren Kette basiert auf IgM-Sequenzen (Subklasse III, da diese die meisten IgM-Sequenzen umfaßt). IgM-Moleküle sind in nicht-aktivierten B-Lymphozyten häufiger vertreten als alle andern Klassen von Immunglobulinen. IgG-Sequenzen überwiegen dagegen in aktivierten B-Lymphozyten, deren Repertoire verschiedener Antikörper sehr viel kleiner ist. Bei einer IgG-Bibliothek bestände außerdem die Gefahr, daß ein oder wenige besonders stark exprimierte IgG die Bibliothek dominieren.

Bis zu 30 Amplifizierungs-Runden wurden zweckmäßigerweise durchgeführt. Die Oligonukleotid-"Primer" enthalten geeignete Restriktionsstellen, um die amplifizierte DNA z.B. in das Antikörper-Expressions-Plasmid pFMT (s.u.) zu insertieren. Dieses Expressions-Plasmid ermöglicht die Expression von Antikörper-cDNA und anschließende Sezernierung der Expressionsprodukte in Bakterien (E.coli). Das Antikörper-Operon des Plasmids enthält die Sequenzen der variablen Teile sowohl der schweren als auch der leichten Kette eines Antikörpers. Geeignete "Leader"-Sequenzen aus dem aminoterminalen Teil eines bakteriellen Proteins ermöglichen die Sezernierung der Antikörperteile. Die "Leader"-Sequenzen werden bei der Sekretion von einem bakteriellen Enzym abgespalten. Während der Sekretion der Antikörper-cDNA-Produkte assoziieren die leichten und schweren Ketten des Antikörpers (mit oder ohne angrenzende konstante Domäne). Dadurch wird ein Antikörper oder Antikörperfragment gebildet, der bzw. das eine funktionelle Antigen-Bindungsstelle enthält. Ähnliche Konstrukte für einzelne Antikörper sind auch von anderen Autoren beschrieben worden (Better et al. (1988), Science 240, 1041, und Skerras & Plückthun (1988), Science 240, 1038).

Die Amplifizierung von DNA, die für die variablen Teile von Antikörpern kodiert, wurde zwar von anderen Autoren beschrieben (Orlandi et al. (1989), Proc. Natl. Acad. Sci. 86, 3833; Sastry et al., (1989) Proc. Natl. Acad. Sci. 86, 5728; Ward et al. (1989), Nature 341, 544); Huse et al. (1989), Science 246, 275). Hier wurde jedoch die u.a. auch für Antikörper kodierende mRNA aus HybridomZellen oder Milz-Lymphozyten nach Behandlung mit einem bestimmten Antigen isoliert. Deshalb wurden dort auch Primer-Sequenzen eingesetzt, die nur auf IgG-Sequenzen basieren. Das ist natürlich dort von Vorteil, wenn möglichst viele Antikörper-DNA-Klone gesucht werden, die von aktivierten Lymphozyten stammen. Mit Primern aus IgG-Sequenzen sind die Chancen viel größer, Klone zu finden, die DNA kodierend für Antikörper gegen das injizierte Antigen enthalten. Hinzuzufügen ist, daß in den vorstehenden Arbeiten murine und damit also nicht-humane Antikörper-DNA synthetisiert und zusätzlich unter Ausschluß von Bereichen der lambda-Kette amplifiziert wurde.

In der vorliegenden Erfindung kommen dagegen Primer-Sequenzen zum Einsatz, die homolog zu Sequenzen in den konstanten Domänen von IgM-cDNA sind. So kann die Erfindung am besten realisiert werden, d.h. eine sehr große Vielfalt an Antikörpern, nämlich das gesamte Antikörper-Repertoire, in Form einer Bibliothek zur Verfügung zu stellen. Die Expression in vorzugsweise E.coli ergibt dann die gesuchte Human-Antikörper-Bibliothek, in der die gesuchten humanen Antikörper bzw. Antikörperteile durch Screenen von bakteriellen Klonen mit dem Antigen der Wahl aufgefunden werden.

In Tab. 1 sind geeignete Oligonukleotidprimer zur Amplifikation zusammengestellt. Die Positionen der vorgenannten Primer auf der µ, kappa- und lambda-Kette sind schematisch in Tab. 2 dargestellt. Die molekularbiologischen Konstruktionen im einzelnen, darunter auch der Expressionsvektor, d.h. das Antikörper-Expressionsplasmid pFMT, sind in den nachfolgenden Beispielen beschrieben. Die Erfindung betrifft folglich Human-Antikörper-Bibliotheken, hergestellt über Transkription der mRNA aus nicht-aktivierten (peripheren) humanen B-Lymphozyten mittels Oligo-dT-Primer, anschließende Amplifikation durch PCR unter Verwendung von Primern enthaltend Sequenzzen homolog zu kpnservierten Bereichen von IgM-cDNA und nachfolgenden Einbau in geeignete Expressionsplasmide zur Expression in Mikroorganismen, vorzugsweise in den Expressionssvektor pFMT zur Expression in E.coli. In einer bevorzugten Ausführungsform wird zusätzlich eine Sequenz eingebaut, die für ein Markerpeptid kodiert, z.B. eine "TAG"-Sequenz, so daß die Expressionsprodukte mit etablierten monoklonalen Antikörpern gegen das Markerpeptid (Wehland et al., (1984), EMBO J. 3, 1295) auf einfache Weise nachgewiesen werden können.

Ferner umfaßt die Erfindung die Verwendung vorstehender Human-Antikörper-Bibliotheken zur Isolierung gesuchter humaner Antikörper bzw. Antikörperteile mit funktioneller, Antigenbindungsstelle durch "Screenen" mit ausgewählten Antigenen sowie Verfahren zur Isolierung der genannten humanen Antikörper oder deren Antigen-bindenden Teile und auch Verfahren zur Herstellung der genannten Human-Antikörper-Bibliotheken.

Die Erfindung umfaßt ferner Expressionsvektoren mit den Eigenschaften des Antikörper-Expressionsplasmids pFMT.

Die nachfolgenden Beispiele führen die Erfindung weiter aus, ohne sie zu begrenzen. Schließlich ist die Erfindung in den Patentansprüchen enthalten.

### Beispiele:

### Beispiel 1: Herstellung eines Antikörper-Expressions-vektors

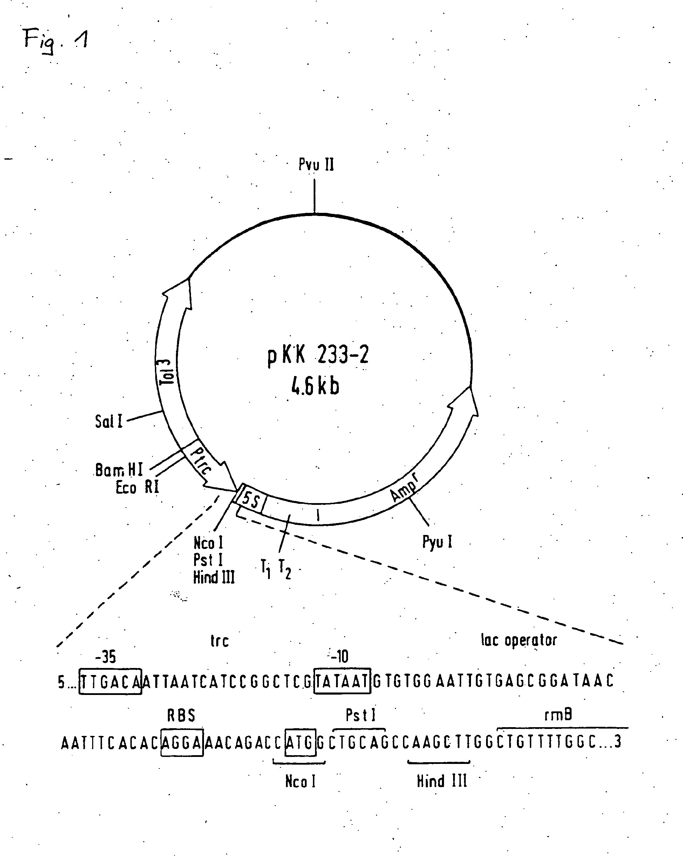

Das Plasmid pKK233-2 (Amann und Brosius, (1985) Gene 40, 183 und Straus und Gilbert, (1985) Proc. Natl. Acad. Sci. 82, 2014) wurde als Basisvektor für den Aufbau des Antikörper-Expressionsvektors gewählt (Fig. 1).

Vor dem Einbau des Antikörper-Operons wurde das Plasmid mit SalI und BamHI geschnitten, die Enden mit Klenow-Polymerase aufgefüllt und ligiert. Dadurch wurden diese beiden Restriktionsstellen und die dazwischenliegende DNA entfernt. Außerdem wurde das Plasmid mit HindIII gespalten, die Enden mit der Klenow-Polymerase aufgefüllt und mit BamHI-Linkern ligiert. Durch diese Operation wurde die HindIII-Restriktionsstelle entfernt und eine BamHI-Stelle insertiert. In dieses modifizierte Plasmid wurde die Antikörper-DNA insertiert. Ein schematischer Konstruktionsweg des Antikörper-Operons, das für eine bicistronische Antikörper-mRNA kodiert, wird in Tab. 3 gezeigt. Um die Sezernierung des Antikörpers zu ermöglichen, wurde die "Leader"-Sequenz des bakteriellen Enzyms Pektatlyase verwendet. Die "Leader"-Sequenz von diesem Enzym ist schon zur Expression und Sezernierung eines chimären Maus-Mensch-Antikörpers (Fab-Fragment, Better et al., a.a.O.) sowie des variablen Teils eines "humanisierten" Antikörpers (Ward et al., a.a.O.; Huse et al., a.a.O.) verwendet worden. DNA für die erste "Leader"Sequenz (P₁ vor der schweren Kette) und die Sequenz für eine zweite Ribosomenbindüngsstelle (RBS) und eine zweite "Leader"-Sequenz (P₂ vor der leichten Kette) wurden aus mehreren Oligonukleotiden synthetisiert (Tab. 4).

Antikörper cDNAs, die für die variable Regionen der schweren und leichten Ketten eines menschlichen Antikörpers (HuVhlys bzw. HuVllys; Riechmann et al., (1988) J. Mol. Biol. 203, 825) kodieren, wurden von Dr. G. Winter (Cambridge, UK) erhalten. Die Restriktionsstellen HindIII (HuVhlys) und EcoRV (HuVllys) wurden eingeführt, um die Insertion der Antikörper-cDNA in den Expressionsvektor zu ermöglichen. Weitere Restriktionsstellen für BanII (HuVhlys) und BstEII bzw. KpnI (HuVllys) wurden eingeführt, um hypervariable Bereiche "en bloc" umzutauschen. Am Ende der HuVhlys-cDNA-Sequenz wurde ein Stopsignal eingebaut. Eine BanII-Stelle in der leichten Kette wurde entfernt. Diese Änderungen wurden mittels "site directed mutagenesis" in dem Bakteriophagen M13mp18 durchgeführt (Zoller und Smith, Meth. Enzymol. 100, 468-500). Die Sequenz der fertigen Antikörper-DNA ist in Tab. 5 gezeigt.

Für die Insertion der "Leader"-Sequenz P₁ (Tab. 4) wurde das modifizierte Plasmid pKK233-2 mit den Restriktionsenzymen NcoI und PstI verdaut und P₁ zwischen diesen Stellen insertiert (pKK233-2-P₁). Weitere Klonierungsschritte bis auf den letzten Schritt wurden mit dem Plasmid pUC18 unternommen. Der Grund besteht darin, daß die Anwesenheit von einzelnen Teilen des Antikörper-Operons in dem Expressionsvektor das Wachstum des bakteriellen Wirts beeinträchtigt.

Vor der Klonierung in pUC18 mußte dessen BamHI-Restriktionsstelle entfernt werden. Nach einem Verdau mit BamH1 wurden die einzelsträngigen Enden mit dem Klenow-Fragment aufgefüllt und religiert. Dieses modifizierte Plasmid wurde dann mit PstI und HindIII verdaut und P₂ plus RBS zwischen diesen Restriktionsstellen einligiert (pUC18-P₂). Bei dieser Operation verschwindet die ursprüngliche HindIII-Restriktionsstelle des Plasmids und eine neue HindIII-Restriktionsstelle wird eingebaut. pUC18-P₂ wurde dann mit PstI und HindIII verdaut und die DNA der schweren Kette (PstI-HindIII-Insert aus M13) wurde in diese beiden Stellen ligiert (pUC18-HP₂). Dieses Plasmid wurde dann mit EcoRV und BamHI verdaut und die DNA der leichten Kette (EcoRV-BamHI-Insert aus M13) wurde hineinligiert (pUCl8-HP₂L).

In einer bevorzugten Ausführungsform wurde in die neue HindIII-Schnittstelle eine "TAG"-Sequenz hineinligiert (Tab. 4). Die "TAG"-Sequenz kodiert für das Peptid Glu-Glu-Gly-Glu-Glu-Phe und wird von dem monoklonalen. Antikörper YL 1/2 (Wehland et al. (1984) EMBO J. 3, 1295) erkannt. Das resultierende Plasmid ist pUC-HTP₂L.

Für die Insertion von HP₂L bzw. HTP₂L in den Expressionsvektor wurden pUC18-HP₂L bzw. pUC-HTP₂L mit PstI und BamHI geschnitten und das betreffende Restriktionsfragment jeweils in das modifizierte Plasmid pKK233-2-P₁ in diese beiden Restriktionsstellen ligiert. Eine schematische Darstellung des fertigen Expressionsvektors pFMT wird in Tab. 6 gezeigt.

### Beispiel 2: Gewinnung von RNA aus menschlichen B-Lymphozyten

Zur Anreicherung peripherer B-Zellen aus menschlichem Blut wurde dieses mit PBS ("phosphate buffered saline") 1:1 verdünnt und über ein Ficoll^{R}(Pharmacia)-Kissen (1,077 kg/l) zentrifugiert. Die Zellen der Interphase wurden mit PBS zweimal gewaschen und in RPMI-Medium, das 10% fötales Kälberserum enthielt, auf einer Plastikunterlage (Kulturflasche) für eine Stunde bei 37°C inkubiert. Die adhärenten Zellen (Monocyten und Makrophagen) heften sich an das Kultur-Gefäß und konnten so aus der Präparation entfernt werden. Die nicht-adhärenten Zellen wurden durch Zentrifugation gesammelt und in 4,4 M Guanidiniumisothiocyanat, 5% Mercaptoethanol und 2% Lauroylsarcosin homogenisiert. Das Homogenat wurde dann über ein Kissen von 5,7 M CsCl für 18 Stunden bei 125000g zentrifugiert. Die sedimentierte RNA wurde in bidest. H₂O gelöst und mit 70% Ethanol und 1/20 Vol. 8M LiCl bei -20°C über Nacht gefällt.

Um eine noch größere Vielfalt von Antikörpern verschiedener Spezifität zu erhalten, wurden RNA-Präparationen von jeweils 500 ml des Blutes von 20 verschiedenen Personen gemischt.

### Beispiel 3: Amplifizierung der Antikörper-DNA

Die mRNA wurde über Oligo-dT-Sepharose (Kit von Phärmacia) gereinigt und mit reverser Transkriptase (Kit von Amersham) und Oligo-dT-Primer in cDNA übersetzt. Die Produkte wurden direkt in der "polymerase chain reaction" (PCR) verwendet. PCR-Primer und Hybridisierungsstellen werden in Tab. 1 bzw. 2 gezeigt. Durch eine Kombination der erhaltenen µ-DNA mit entweder kappa- oder lambda-DNA im Vektor pFMT wurden zwei verschiedene Expressionsbanken hergestellt. Die Verwendung verschiedener Primer für die Synthese der nicht-kodierenden Stränge in der "polymerase chain reaction" ermöglicht die Herstellung zweier verschiedener Antikörper-Typen, die in einem Fall nur die variable Domäne, im anderen Fall noch zusätzlich eine konstante Domäne enthalten (dem Fab-Fragment eines Antikörpers ähnlich). Für die PCR wurden 4 µl einer cDNA Synthese mit jeweils 0,2 nmol der zwei Primer in einem Volumen von 50 µl umgesetzt. Der Reaktionsansatz enthielt 100 mM KCl, 0,1% Gelatine und 2,5 U Taq-Polymerase. Nach 30 Polymerisationszyklen, bestehend aus 1 Min. 95°C, 2 Min. 55°C und 2 Min. 72°C wurde die DNA mit Ethanol gefällt.

### Beispiel 4: Insertion der Antikörper-DNA in das Expressionsplasmid

Die gefällte DNA wurde in Ladepuffer für Agarosegele (0.1% Bromphenolblau, 7% Ficoll^{R} [Pharmacia]) aufgenommen und über 2% Agarose bei 10V/cm in TBE-Puffer (45 mM Tris-Borat pH 8.0, 10 mM EDTA) aufgetrennt. Die synthetisierte Antikörper-DNA wurde anhand ihres Molekulargewichtes identifiziert und aus dem Gel eluiert. Nach Fällung mit Ethanol wurde sie in Puffer für die jeweiligen Restriktionsenzyme aufgenommen und mit den entsprechenden (vgl. Tab. 1 und 2) Restriktionsenzymen (Boehringer Mannheim) geschnitten . Nach einer Fällung in Ethanol wurde sie in den ebenso geschnittenen Vektor pFMT einligiert, wie in der Tab. 7 schematisch gezeigt.

### Beispiel 5: Expression und "Screenen" von Antikörpern in E.coli

Kompetente E.coli werden mit pFMT-Plasmiden., die die insertierte Antikörper-DNA-Bibliothek enthalten, transfiziert, auf Agaroseplatten aufgezogen und anschließend mit Nitrozellulosefiltern inkubiert, die mit dem gesuchten Antigen beschichtet sind. Nach der Entfernung unspezifisch gebundener Antikörper werden die aktiven Klone mit einem markierten Antikörper gegen die aus E.coli sezernierten menschlichen Immunglobuline identifiziert. In der bevorzugten Ausführungsform wird zur Identifizierung der gesuchten Klone der monoklonale Antikörper YL 1/2 verwendet, der gegen die "TAG"-Sequenz gerichtet ist.

### Legende zu Fig. 1:

Restriktionskarte des Expressionsvektors pKK233-2 (Amann und Brosius, a.a.O.)
Ptrc bedeutet hybrider Tryptophan-lac Promotor
RBS bedeutet Ribosomenbindungsstelle
rrnB bedeutet ribosomale RNA B (5S RNA)
5S bedeutet Gen für 5S RNA (enthält rrnB)

Vor der Klonierung von Antikörper-DNA in den Expressionsvektor wurden die folgenden Änderungen durchgeführt:
1) Die SalI und EcoRI Restriktionsstellen wurden zusammen mit der zwischenliegenden DNA entfernt.
2) Die HindIII Restriktionsstelle wurde in eine BamHI Restriktionsstelle umgewandelt.

**Tab. 1 Oligonukleotid-"Primer" für die Amplifikation von cDNA mit der "Polymerase chain reaction".**

| |
|---|
| 1. Oligonukleotid-"Primers" für die Vorwärts-PCR |
| D. µ-Kette |
| |
| E. κ-Kette |
| |
| F. λ-Kette |
| |
| 2. Oligonukleotid-"Primers" für die Rückwärts-PCR ( Variable Domäne plus angrenzende konstante Domäne) |
| A1. µ-Kette (ohne "TAG-'Sequenzen) |
| |
| A2. µ-Kette (mit "TAG-'Sequenzen) |
| |
| B. κ-Kette |
| |
| C. λ-Kette |
| |
| 3. Oligonukleotid-"Primers" für die Rückwärts-PCR (Variable Domäne). |
| G1. µ-Kette (ohne "TAG'-Sequenzen) |
| |
| G2. µ-Kette (mit "TAG''-Sequenzen) |
| |
| H. κ-Kette |
| |
| I.λ-Kette |
| |

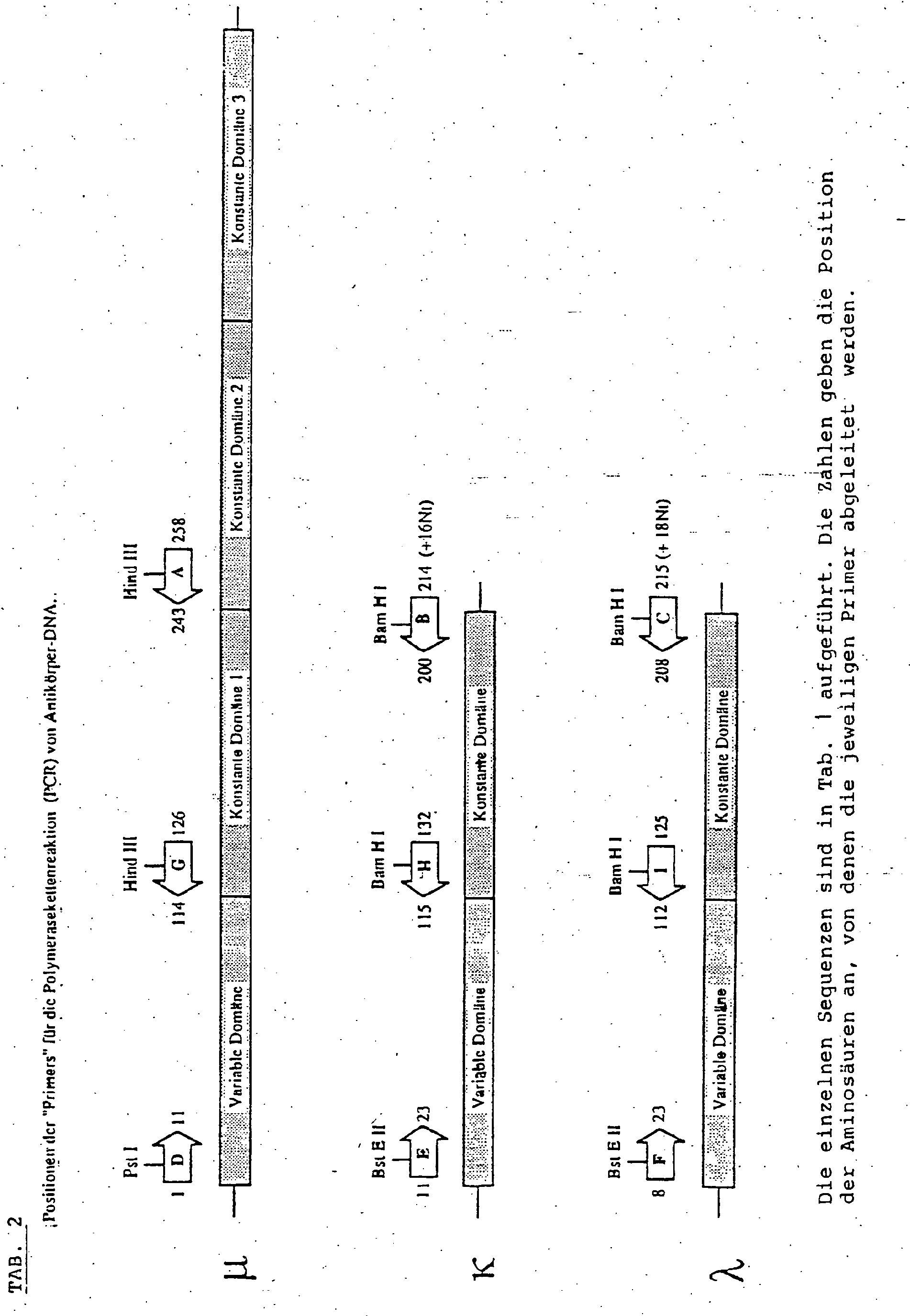

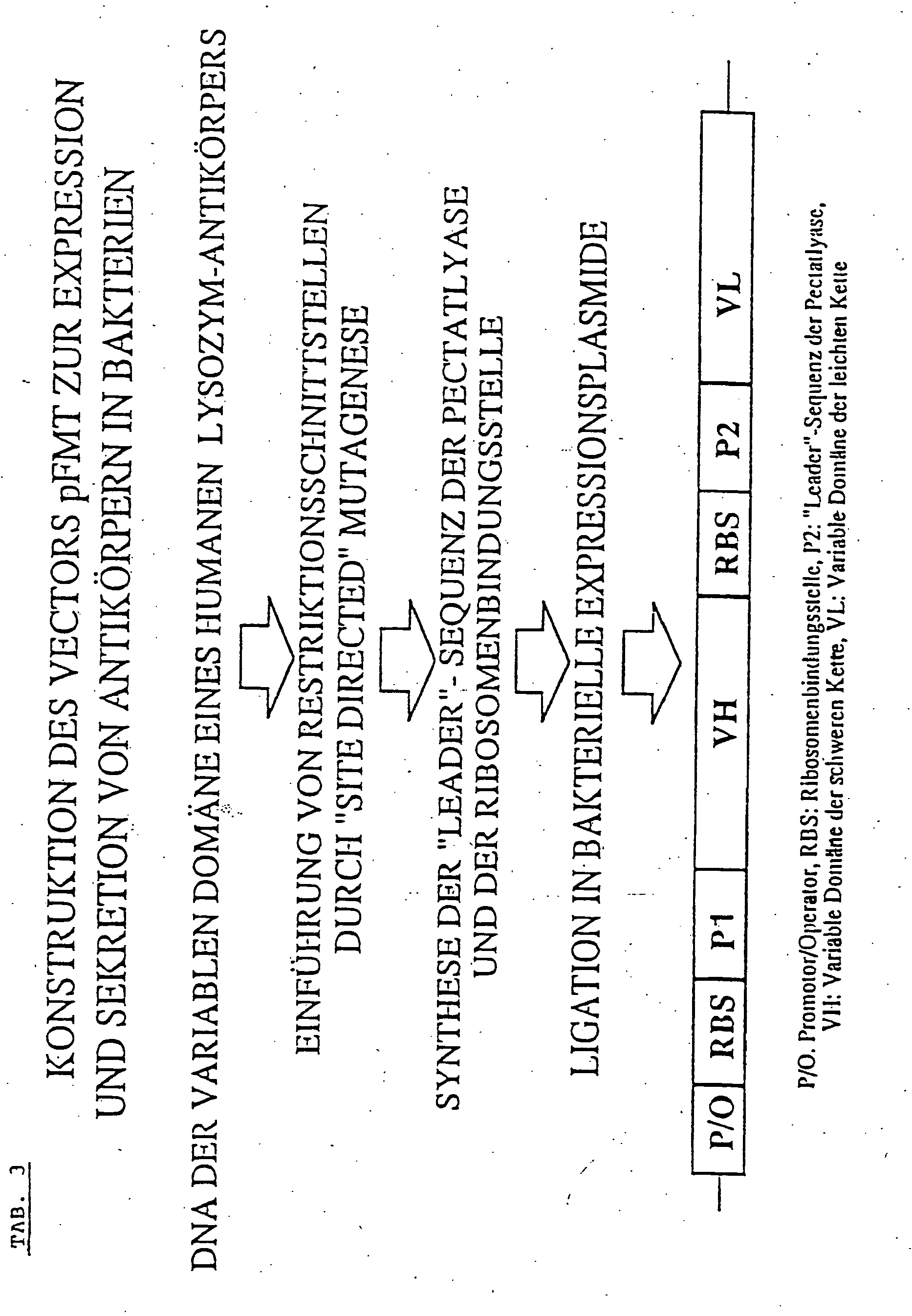

**Tab. 4 Sequenzen der "Leader"-Sequenzen P1 und P2 im Antikörper-Operon sowie der "Tag"-Sequenzen**

| |
|---|
| **P1** |
| |
| |
| **P2** |
| |
| Die Nukleotide in Kammern sind die angrenzenden Nukleotide des Plasmids. |
| |
| Die "Leader"-Sequenzen wurden durch die Hybridisierung der folgenden Oligonukleotide synthetisiert. |
| |
| **P1** |
| a.5 'CATGAAATACCTCTTGCCTACGGCAGCCGCTGGCTTG3' SEQ ID NO: 16 |
| b.3'TTTATGGAGAACGGATGCCGTCGGCGACCGAACGACGACGACCGTCGAGTCGGCCGCTACCGCGTTCAAGTCGS' SEQ ID NO: 17 |
| c. 5'CTGCTGCTGGCAGCTCAGCCGGCGATGGCGCAAGTTCAGCTGCA3' SEQ ID NO: 18 |
| |
| **P2** |
| a. 5' GCCAAGCTTGAATTCATTAAAGAGGAGAAA3' SEQ ID NO : 19 |
| b.5'TTAACTCCATGAAGTACTTACTGCCGACCGCTGCG3' SEQ ID NO: 20 |
| |
| d.5'GCTCAGCCGGCTATGGCTGATATCGGATCC3' SEQ ID NO: 22 |
| e.5'GCGGGTCTCCTC-CTGTTGGCG3' SEQ ID NO: 23 |
| |
| Die Tag-Sequencen wurden durch die Hybridisierung der folgenden Sequenzen synthetisiert: |
| a.5'AGCTTGAAGAAGGTGAAGAATTCTAATG3' SEQ ID NO: 24 |
| b.5'AGCTCATTAGAATTCTTCACCTTCA3' SEQ ID NO: 25 |

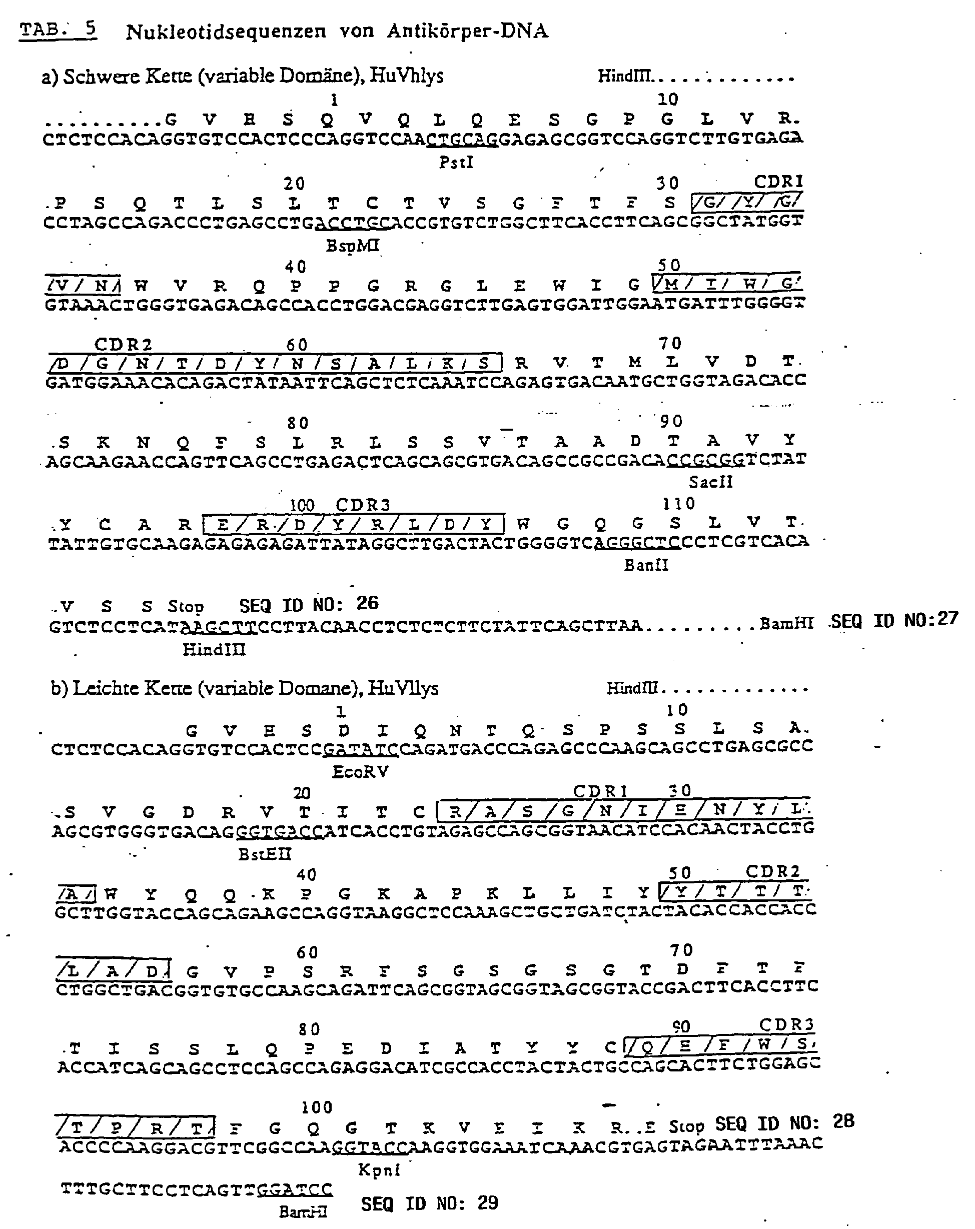

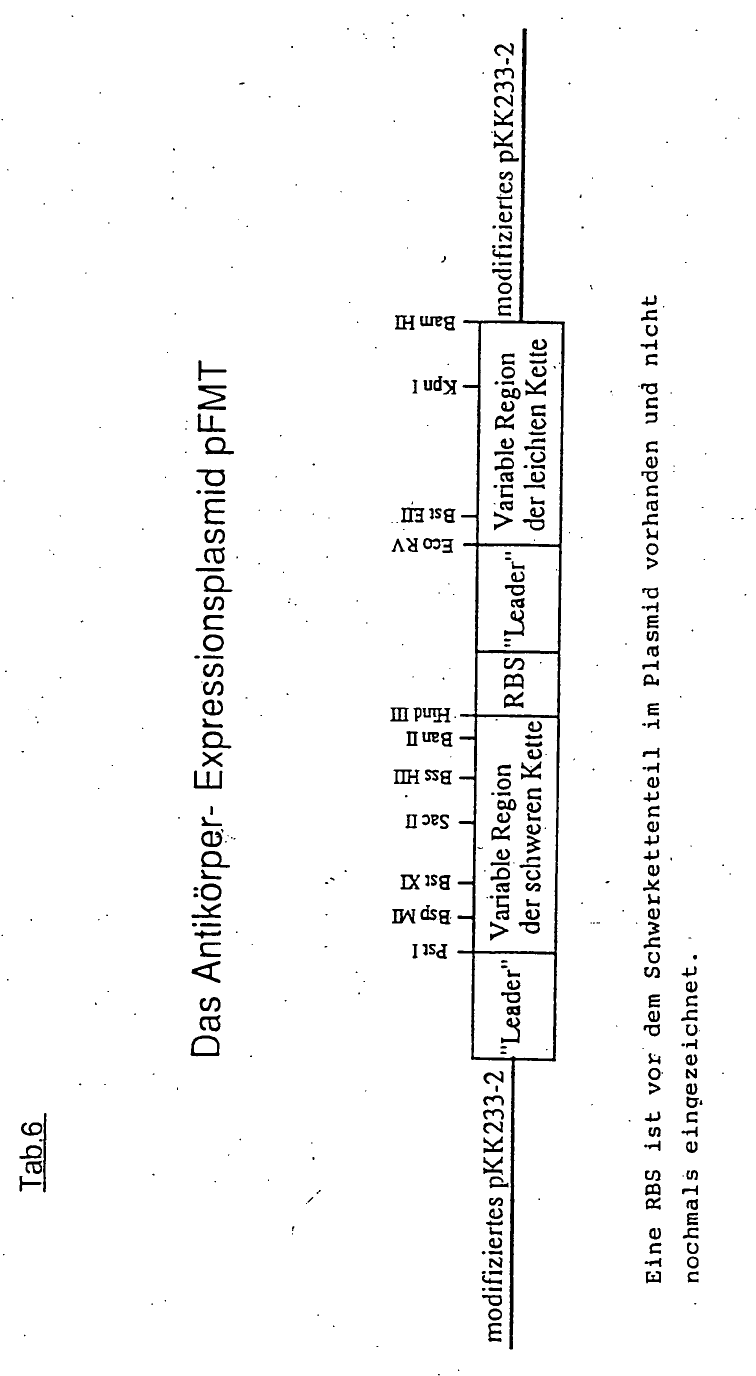

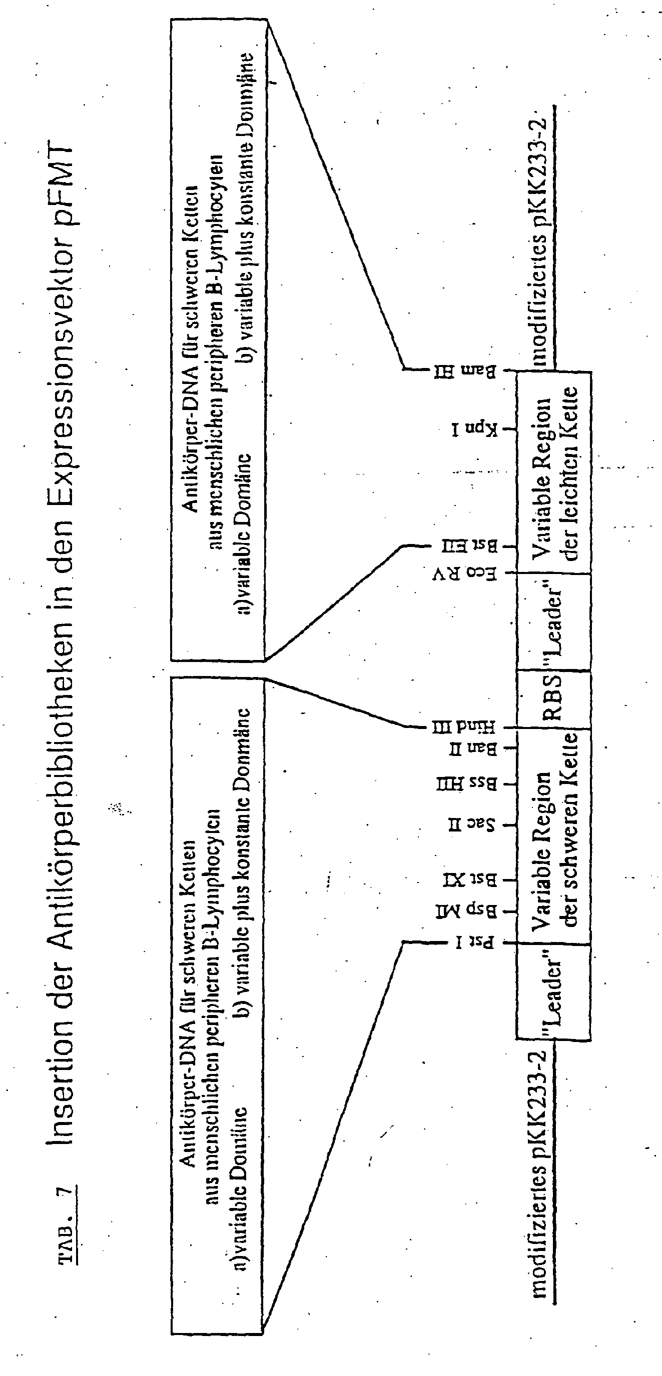

## Patentansprüche (Patentansprüche für folgende(n) Vertragsstaat(en): AT, BE, CH, DE, DK, FR, GB, GR, IT, LI, LU, NL, SE)

1. Human-Antikörper-Bibliotheken, erhältlich über Transskription isolierter mRNA aus nicht aktivierten peripheren humanen B-Lymphozyten in cDNA, anschließende Amplifikation von für Antikörper kodierender cDNA durch "Polymerase-Chain-Reaction" (PCR) mittels geeigneter Primer, nachfolgenden Einbau in geeignete Expressionsplasmide und Expression der darin enthaltenen, in cDNA amplifizierten relevanten Antikörper-RNA in Einzelklonen, **dadurch gekennzeichnet, daß** der Entwurf der Primer für die Rückreaktion zur Synthese des nicht-kodierenden Stranges der DNA der schweren Kette auf IgM-Sequenzen basiert und die Verwendung verschiedener Primer für die Synthese des nicht-kodierenden Stranges die Herstellung eines Antikörpertyps ermöglicht, der aus den variablen Domänen und den ersten konstanten Domänen besteht.

2. Human-Antikörper-Bibliotheken nach Anspruch 1, **dadurch gekennzeichnet, daß** die Expression im Plasmid pFMT gemäß Tab. 6 erfolgt.

3. Verfahren zur Herstellung von Human-Antikörper-Bibliotheken, **dadurch gekennzeichnet, daß** mRNA aus nicht aktivierten peripheren humanen B-Lymphozyten isoliert und in cDNA überschrieben wird, anschließend die für Antikörper kodierende cDNA durch PCR mittels geeigneter Primer amplifiziert wird, darauf folgend ein Einbau in geeignete Expressionsplasmide vorgenommen wird und schließlich Expression der Antikörper-cDNA in Einzelklonen erfolgt, **dadurch gekennzeichnet, daß** der Entwurf der Primer für die Rückreaktion zur Synthese des nicht-kodierenden Stranges der DNA der schweren Kette auf IgM-Sequenzen basiert und daß durch die Verwendung verschiedener Primer für die Synthese des nicht-kodierenden Stranges die Herstellung eines Antikörpertyps ermöglicht wird, der aus den variablen Domänen und den ersten konstanten Domänen besteht.

4. Verfahren nach Anspruch 4, **dadurch gekennzeichnet, daß** die Expression im Plasmid pFMT gemäß Tab. 6 erfolgt.

5. Verfahren zur Isolierung von spezifischen humanen Antikörperfragmenten, die jeweils aus den variablen Domänen und den ersten konstanten Domänen bestehen, **dadurch gekennzeichnet, daß** Human-Antikörper-Bibliotheken nach Anspruch 1, 2 oder 3 mit spezifischen Antigenen durchsucht werden.

6. Verwendung von Human-Antikörper-Bibliotheken nach Anspruch 1, 2 oder 3 zur Isolierung von spezifischen humanen Antikörperfragmenten.

## Patentansprüche (Patentansprüche für folgende(n) Vertragsstaat(en): ES)

1. Verfahren zur Herstellung von Human-Antikörper-Bibliotheken; **dadurch gekennzeichnet, daß** mRNA aus nicht aktivierten peripheren humanen B-Lymphozyten isoliert und in cDNA überschrieben wird, anschließend die für Antikörper kodierende cDNA durch PCR mittels geeigneter Primer amplifiziert wird, darauf folgend ein Einbau in geeignete Expressionsplasmide vorgenommen wird und schließlich Expression der Antikörper-cDNA in Einzelklonen erfolgt, **dadurch gekennzeichnet, daß** der Entwurf der Primer für die Rückreaktion zur Synthese des nicht-kodierenden Stranges der DNA der schweren Kette auf IgM-Sequenzen basiert und daß durch die Verwendung verschiedener Primer für die Synthese des nicht-kodierenden Stranges die Herstellung eines Antikörpertyps ermöglicht wird, der aus den variablen Domänen und den ersten konstanten Domänen besteht.

2. Verfahren nach Anspruch 1, **dadurch gekennzeichnet, daß** die Expression im Plasmid pFMT gemäß Tab. 6 erfolgt.

3. Verfahren zur Isolierung von spezifischen humanen Antikörperfragmenten, **dadurch gekennzeichnet, daß** Human-Antikörper-Bibliotheken, hergestellt nach Anspruch 1 oder 2, mit spezifischen Antigenen durchsucht werden.

## Claims (Claims for the following Contracting State(s): AT, BE, CH, DE, DK, FR, GB, GR, IT, LI, LU, NL, SE)

1. A human-antibody library obtainable by means of transcription of isolated mRNA from nonactivated peripheral human B-lymphocytes into cDNA, subsequent amplification of cDNA coding for antibodies by polymerase chain reaction (PCR) by means of suitable primers, subsequent incorporation into suitable expression plasmids and expression, in individual clones, of the relevant antibody RNA which is contained therein and has been amplified in cDNAs, wherein the design of the primers for the reverse reaction for the synthesis of the noncoding strand of the DNA of the heavy chain is based on IgM sequences and the use of different primers for the synthesis of the noncoding strand makes possible the preparation of an antibody type which consists of the variable domains and first constant domains.

2. A human-antibody library as claimed in claim 1, wherein the expression takes place in the plasmid pFMT according to Tab. 6.

3. A process for preparing a human-antibody library, which comprises mRNA being isolated from nonactivated peripheral human B-lymphocytes and being transcribed into cDNA, subsequently amplifying the cDNA coding for antibodies by PCR by means of suitable primers, then carrying out an incorporation into suitable expression plasmids and finally expressing the antibody cDNA in individual clones, wherein the design of the primers used for the reverse reaction for the synthesis of the noncoding strand of the DNA of the heavy chain is based on IgM sequences and the use of different primers for the synthesis of the noncoding strand makes possible the preparation of an antibody type which consists of the variable domains and first constant domains.

4. The process as claimed in claim 3, wherein the expression takes place in plasmid pFMT according to Tab. 6.

5. A process for isolating specific human antibody fragments which each consist of the variable domains and the first constant domains, comprising screening human-antibody libraries as claimed in claim 1, 2 or 3 using specific antigens.

6. The use of a human-antibody library as claimed in claim 1, 2 or 3 for isolating specific human antibody fragments.

## Claims (Claims for the following Contracting State(s): ES)

1. A process for preparing a human-antibody library, which comprises mRNA being isolated from nonactivated peripheral human B-lymphocytes and being transcribed into cDNA, subsequently amplifying the cDNA coding for antibodies by PCR by means of suitable primers, then carrying out an incorporation into suitable expression plasmids and finally expressing the antibody cDNA in individual clones, wherein the design of the primers used for the reverse reaction for the synthesis of the noncoding strand of the DNA of the heavy chain is based on IgM sequences and the use of different primers for the synthesis of the noncoding strand makes possible the preparation of an antibody type which consists of the variable domains and first constant domains.

2. The process as claimed in claim 1, wherein the expression takes place in plasmid pFMT according to Tab. 6.

3. A process for isolating specific human antibody fragments, comprising screening human-antibody libraries as claimed in claim 1 or 2 using specific antigens.

## Revendications (Revendications pour l'(les) Etat(s) contractant(s) suivant(s): AT, BE, CH, DE, DK, FR, GB, GR, IT, LI, LU, NL, SE)

1. Bibliothèque d'anticorps humains pouvant être obtenue par transcription en ADNc d'ARNm provenant de lymphocytes B humains, périphériques, non activés, par amplification ensuite d'ADNc codant pour des anticorps « polymerase-Chain-reaction » (PCR) au moyen d'amorces appropriées, par incorporation ensuite dans des plasmides d'expression appropriés et expression des anticorps-ARN pertinents amplifiés dans de l'ADNc, qui y sont contenus, en des clones individuels, **caractérisée en ce que** le plan de l'amorce pour la réaction en retour pour la synthèse du brin non codant de l'ADN de la chaîne lourde repose sur des séquences IgM et l'utilisation de diverses amorces pour la synthèse du brin non codant rend possible la préparation d'un type d'anticorps qui est constitué des domaines variables et des premiers domaines constants.

2. Bibliothèque d'anticorps humains suivant la revendication 1, **caractérisée en ce que** l'expression dans le plasmide pFMT s'effectue suivant le tableau 6.

3. Procédé de préparation de bibliothèques d'anticorps humains, **caractérisé en ce que** l'on isole de l'ARNm de lymphocytes B humains, périphériques, non activés et on le transcrit en de l'ADNc, on amplifie ensuite l'ADNc codant pour les anticorps par PCR au moyen d'amorces appropriées, puis on effectue une incorporation dans des plasmides d'expression appropriés et on effectue enfin une expression de l'anticorps - ADNc en des clones individuels, **caractérisée en ce que** le plan des amorces pour la réaction en retour de synthèse du brin non codant de l'ADN de la chaîne lourde repose sur des séquences IgM et **en ce que**, par l'utilisation de diverses amorces pour la synthèse du brin non codant, on rend possible la préparation d'un type d'anticorps qui est constitué des domaines variables et des premiers domaines constants.

4. Procédé suivant la revendication 3, **caractérisé en ce que** l'expression dans le plasmide pFMT s'effectue suivant le tableau 6.

5. Procédé pour isoler des fragments d'anticorps humain spécifiques qui sont constitués respectivement des domaines variables et des premiers domaines constants, **caractérisé en ce que** l'on analyse des bibliothèques d'anticorps humains suivant la revendication 1, 2 ou 3 avec des antigènes spécifiques.

6. Utilisation de bibliothèques d'anticorps humains suivant la revendication 1, 2 ou 3, pour l'isolation de fragments d'anticorps humains spécifiques.

## Revendications (Revendications pour l'(les) Etat(s) contractant(s) suivant(s): ES)

1. Procédé de préparation de bibliothèques d'anticorps humains, **caractérisé en ce que** l'on isole de l'ARNm de lymphocytes B humains, périphériques, non activés et on le transcrit en de l'ADNc, on amplifie ensuite l'ADNc codant pour les anticorps par PCR au moyen d'amorces appropriées, puis on effectue une incorporation dans des plasmides d'expression appropriés et on effectue enfin une expression de l'anticorps - ADNc en des clones individuels, **caractérisée en ce que** le plan des amorces pour la réaction en retour de synthèse du brin non codant de l'ADN de la chaîne lourde repose sur des séquences IgM et **en ce que**, par l'utilisation de diverses amorces pour la synthèse du brin non codant, on rend possible la préparation d'un type d'anticorps qui est constitué des domaines variables et des premiers domaines constants.

2. Procédé suivant la revendication 1, **caractérisé en ce que** l'expression dans le plasmide pFMT s'effectue suivant le tableau 6.

3. Procédé pour isoler les fragments d'anticorps humain spécifiques, **caractérisé en ce que** l'on analyse des bibliothèques d'anticorps humains suivant la revendication 1, ou 2 avec des antigènes spécifiques.
